# EUROPÄISCHE PATENTSCHRIFT

(11) **EP 0 693 950 B1**
(45) Veröffentlichungstag und Bekanntmachung des Hinweises auf die Patenterteilung: **06.08.1997**
(21) Anmeldenummer: 94912446.5
(22) Anmeldetag: 09.04.1994
(51) Int. Cl.: A61M 16/00

(54) **EINRICHTUNG ZUM BEATMEN EINES UNFALLOPFERS**
RESPIRATORY DEVICE FOR AN ACCIDENT VICTIM
DISPOSITIF PERMETTANT DE PRATIQUER LA RESPIRATION ARTIFICIELLE A UN ACCIDENTE

(30) Priorität: 14.04.1993 DE 4312215; 24.12.1993 DE 4344403
(43) Veröffentlichungstag der Anmeldung: 31.01.1996
(73) Patentinhaber: WEIDINGER, Max, D-96450 Coburg (DE)
(72) Erfinder: WEIDINGER, Max, D-96450 Coburg (DE)
(74) Vertreter: LOUIS, PÖHLAU, LOHRENTZ & SEGETH
(86) Internationale Anmeldenummer: DE9400403
(87) Internationale Veröffentlichungsnummer: WO9423779

(56) Entgegenhaltungen:
- EP-A- 0 265 163
- WO-A-88/04187
- WO-A-93/08860
- GB-A- 904 814
- US-A- 3 802 428
- US-A- 4 662 367
- US-A- 5 020 529

## Beschreibung

Die Erfindung betrifft eine Einrichtung zum Beatmen insbes. eines Unfallopfers, gemäß dem Oberbegriff des Anspruchs 1.

Eine Einrichtung zum Beatmen eines Unfallopfers ist aus der US 35 38 913 bekannt. Diese bekannte Einrichtung weist einen Tubus auf, der in seiner Längsrichtung teleskopartig zusammenschiebbar, d.h. verkürzbar, ist. Die minimalen axialen Abmessungen sind dort durch das eine oder andere Teleskopelement des Tubus festgelegt. Im Querschnitt ist der Tubus dieser bekannten Einrichtung nicht veränderbar, so daß diese Einrichtung einen nicht unerheblichen Platzbedarf besitzt. Zusammenfaltbar, d.h. an den Tubus anlegbar, sind dort nur zwei Flügelelemente am Tubus vorgesehen, um im auseinandergeklappten Zustand einen Berührungsschutz zu bilden. Die beiden Öffnungsränder des Tubus sind bei dieser bekannten Einrichtung einfach kreisrund geformt und mit einem Wulst ausgebildet. Eine Orientierung wird bei dieser Einrichtung nur durch die beiden auf- bzw. zusammenklappbaren Flügelelemente bewirkt.

Eine Einrichtung der eingangs genannten Art ist aus der FR 14 51 616 bekannt. Ein Filterelement aus elastisch nachgiebigem Schaumstoffmaterial vorzusehen, das nicht nur eine entsprechende Filterwirkung ergibt, sondern in vorteilhafter Weise gleichzeitig auch als Federelement dient, mit dessen Hilfe der Tubus von der zusammengefalteten Ruhestellung in die aktive Betriebsstellung auf-. bzw. auseinandergefaltet wird, ist dort nicht vorgesehen. Vielmehr ist anstelle eines Filterelementes aus elastischem Material nur eine in den Mund des Unfallopfers einzusteckende Leitung vorgesehen.

Das DE-GM 87 09 867 zeigt eine ähnliche Einrichtung, die jedoch keinen zusammenfaltbaren Tubus aufweist. Vielmehr besteht der Tubus bei dieser bekannten Einrichtung aus einem formstabilen Material, so daß diese bekannte Einrichtung einen bestimmten Platz- bzw. Raumbedarf aufweist. Außerdem besteht diese Einrichtung aus einer Anzahl Einzelteile, die zur Beatmungseinrichtung zusammengebaut werden müssen, was sich auf den Anschaffungspreis der Einrichtung auswirkt. Eine solche Einrichtung ist demzufolge vor allem für den professionellen Einsatz bei Notärzten, bei der Rettung, bei der Feuerwehr o.dgl. vorgesehen.

Die FR-1 600 919 offenbart eine zum Beatmen eines Unfallopfers vorgesehene Einrichtung, welche aus zwei Teilen zusammengesetzt ist, die miteinander gelenkig verbunden sind.

Diese bekannte Einrichtung ist mit einem formstabilen auswechselbaren Filterelement versehen.

Eine der zuletzt genannten Einrichtung ähnliche Einrichtung aus zwei Teilen und einem zwischen diesen anordenbaren bzw. angeordneten Filterelement ist aus der FR 26 24 025 bekannt. Auch hier ist das Filterelement formstabil ausgebildet.

Der Erfindung liegt die Aufgabe zugrunde, eine Einrichtung der eingangs genannten Art zu schaffen, die sich zur platzsparenden Unterbringung in einem "Erste Hilfe"-Verbandkasten eines Kraftfahrzeugs oder eines Betriebes eignet und bei der die Mundstücke des Tubus derartig geformt sind, daß eine optimale Anordnung des Tubus zwischen zu beatmendem Unfallopfer und Helfer und somit eine optimale Beatmung eines Unfallopfers möglich ist, wobei ein Blut- bzw. Speichelfluß durch den Tubus verhindert wird.

Diese Aufgabe wird erfindungsgemäß durch die im Patentanspruch 1 dazu angegebenen Merkmale gelöst. Bevorzugte Aus- und Weiterbildungen der erfindungsgemäßen Einrichtung sind in den Unteransprüchen gekennzeichnet.

Die erfindungsgemäße Einrichtung ist einfach herstellbar, was sich auf die Herstellungskosten günstig auswirkt. Sie ist ein kostengünstiger Massenartikel, der sich gut zur Bestückung eines "Erste Hilfe"-Verbandkastens eignet. Außerdem bietet sie einen guten hygienischen Schutz des Helfers, so daß dieser seiner Helferpflicht sicher nachkommen kann.

Dadurch, daß die beiden Öffnungsränder des Tubus derartig gestaltet sind, daß sie im auseinandergefalteten Aktivzustand der Einrichtung zueinander senkrecht orientierte Mundstücke bilden, ist es möglich, die Einrichtung bzw. den Tubus relativ kurz zu gestalten, weil der Mund des Helfers zum Mund des Unfallopfers senkrecht orientiert sein kann, so daß sich die Nasen des Helfers und des Unfallopfers nicht behindern. Andererseits weist der Tubus selbstverständlich axiale Abmessungen auf, die ausreichend lang sind, um eine Berührung zwischen Unfallopfer und Helfer zuverlässig auszuschließen.

Durch die erfindungsgemäße Ausbildung der Einrichtung derart, daß die Mundstückstruktur des Helfer-Öffnungsrandes des Tubus konkav gekrümmt eingezogen und die Mundstückstruktur des Opfer-Öffnungsrandes des Tubus mit zwei Mundwinkel-Ansätzen ausgebildet ist, ergibt sich der Vorteil, daß im Aktivzustand, d.h. im auseinandergefalteten Zustand der Einrichtung die beiden Mundwinkel-Ansätze voneinander im Mund des Unfallopfers beabstandet, d.h. in den beiden Mundwinkeln des Unfallopfers passend positioniert werden, so daß eine sichere Beatmung des Unfallopfers möglich ist. Das bedeutet jedoch eine gute Wirksamkeit der Einrichtung, d.h. die Möglichkeit einer zuverlässigen Beatmung insbes. eines Unfallopfers.

Das bei der erfindungsgemäßen Einrichtung im Inneren des Tubus vorgesehene Filterelement aus einem luftdurchlässigen, elastisch nachgiebigem, offenporigen Schaumstoffmaterial bildet nicht nur eine Sperre, um einen Blut- bzw. Speichelfluß durch den Tubus hindurch zuverlässig auszuschließen, ohne jedoch die Beatmungs-Luftströmung durch den Tubus hindurch zu beeinträchtigen, sondern das elastisch nachgiebige Schaumstoffmaterial bildet gleichzeitig auch quasi ein Federelement, durch das eine mehr oder weniger automatische Verstellung der Einrichtung in den auseinandergefalteten Aktivzustand erfolgt, sobald die Einrichtung z.B. aus einem Verbandkasten entnommen wird.

Bei der erfindungsgemäßen Beatmungseinrichtung kann das Filterelement in den Tubus eingeschweißt, eingeklebt oder auf eine andere bekannte Art und Weise im Tubus befestigt sein. Einer sicheren, zuverlässigen Befestigung des Filterelementes im Tubus ist es dienlich, wenn der Tubus in seinem Inneren mit einer Halterung für das Filterelement ausgebildet ist. Diese Halterung kann erfindungsgemäß von zwei Ringwülsten gebildet sein, die in Längsrichtung des Tubus voneinander beabstandet sind. Die besagten Ringwülste werden zweckmäßigerweise mit dem Tubus einteilig, d.h. im selben Formgebungs-Arbeitsgang hergestellt. Der Abstand der beiden Ringwülste ist hierbei an die Dicke des Filterelementes angepaßt, so daß es einfach möglich ist, das Filterelement zwischen den beiden Ringwülsten einzuklemmen. Das bedeutet eine einfache Montage des Filterelementes und bei entsprechender Dimensionierung der Ringwülste eine sichere Befestigung des Filterelementes im Tubus der erfindungsgemäßen Beatmungseinrichtung.

Eine noch weiter verbesserte Festlegung des Filterelementes im Tubus wird erzielt, wenn erfindungsgemäß die Halterung von einem den Tubus querenden Gitterelement und einem davon in Längsrichtung des Tubus beabstandeten Ringwulst gebildet ist, wobei das Gitterelement auf der dem Opfer-Öffnungsrand zugewandten Seite und der Ringwülst auf der dem Helfer-Öffnungsrand zugewandten Seite des Tubus vorgesehen ist. Auch bei einer solchen Ausbildung der Beatmungseinrichtung ist der Abstand zwischen dem Gitterelement und dem Ringwulst an die Dicke des Filterelementes angepaßt. Das Filterelement liegt im in den Tubus eingebauten Zustand also am Gitterelement zumindest dann an, wenn die Beatmungseinrichtung zum Beatmen eines Opfers, insbes. Unfallopfers, angewandt wird. Das Gitterelement und der davon beabstandete Ringwulst werden zweckmäßigerweise gleichzeitig bei der Formung des normalerweise aus einem geeigneten Kunststoffmaterial bestehenden Tubus, d.h. mit diesem einteilig, realisiert. Durch das dem Opfer-Öffnungsrand zugewandte Gitterelement wird in jedem Fall zuverlässig verhindert, daß beispielsweise während des Beatmungsvorgangs unbeabsichtigt das Filterelement in den Mund des zu beatmenden Opfers, insbes. Unfallopfers, hineingeblasen wird.

Um den Tubus der erfindungsgemäßen Einrichtung zwischen der platzsparend zusammengefalteten Ruhe- bzw. Lagerposition und der auseinandergefalteten Aktivstellung der Einrichtung problemlos verändern zu können, hat es sich als vorteilhaft erwiesen, wenn der Tubus mit Faltlinien ausgebildet ist. Bei diesen Faltlinien kann es sich um linienförmige Schwachstellen im Material des Tubus, um linienförmige Schwachstellen in Form sog. Kunststoff-Filmscharniere o.dgl. handeln. Diese Faltlinien legen nicht nur die platzsparende Ruhe- bzw. Lagerposition der Einrichtung fest, sondern auch deren auseinandergefalteten Aktivzustand, in welchem dann die beiden Mundstücke des Tubus zueinander senkrecht orientiert sind.

Eine weiter verbesserte Ausbildung der erfindungsgemäßen Einrichtung ist dadurch gekennzeichnet, daß das Filterelement ein Atemröhrchen aufweist, das vom Filterelement zum Opfer-Öffnungsrand weist. Dieses Atemröhrchen dient insbes. dazu, zwischen die oberen und unteren Schneidezähne des Unfallopfers eingesteckt zu werden, um die Beatmung des Unfallopfers zuverlässig durchführen zu können. Dieses Atemröhrchen kann aus einem Kunststoffmaterial oder bspw. auch aus einem Pappkartonmaterial entsprechender Festigkeit bestehen. Die Wahl der Materialien für die erfindungsgemäße Einrichtung ist auch davon abhängig, ob die Einrichtung zum einmaligen oder zum mehrfachen Gebrauch geeignet sein soll.

Die Beatmung eines Unfallopfers kann in vorteilhafter Weise dadurch weiter verbessert werden, daß bei der erfindungsgemäßen Einrichtung der Tubus mit einem Nasenabdeckorgan versehen ist. Dieses kann mit einem Polsterelement ausgebildet sein, um eine Abdichtung der Nasenöffnungen des Unfallopfers zu bewirken. Das besagte Polsterelement kann wie das weiter oben erwähnte Filterelement aus einem weichen nachgiebigen Schaumstoffmaterial bestehen.

Eine herstellungstechnisch einfache Ausbildung wird bei einer Beatmungseinrichtung der zuletzt beschriebenen Art, d.h. bei einer Beatmungseinrichtung mit einem Nasenabdeckorgan, erreicht, wenn das Nasenabdeckorgan an den Tubus einteilig angeformt ist. Eine solche Ausbildung weist den Vorteil auf, daß ein Manipulationsaufwand zur Anbringung des Nasenabdeckorganes am Tubus der erfindungsgemäßen Beatmungseinrichtung nicht gegeben ist. Das einteilig vom Tubus wegstehende Nasenabdeckorgan führt jedoch dazu, daß die Beatmungseinrichtung insgesamt einen bestimmten Platzbedarf besitzt.

Eine platzsparende Ausbildung der mit einem Nasenabdeckorgan versehenen Einrichtung ergibt sich, wenn das Nasenabdeckorgan mit dem Tubus schwenkbeweglich verbunden ist. Hierbei ist das Nasenabdeckorgan zwischen einer mit dem Tubus fluchtend anliegenden Ruheposition und einer vom Tubus zumindest annähernd senkrecht wegstehenden Aktivstellung verschwenkbar. Demselben Zweck, d.h. einer platzsparenden Unterbringung der erfindungsgemäßen Einrichtung bspw. in einem Verbandkasten ist es dienlich, wenn bei einer Einrichtung der zuletzt genannten Art das Nasenabdeckorgan mit Faltlinien ausgebildet ist.

Insgesamt ergibt sich also eine Einrichtung zum Beatmen insbes. eines Unfallopfers, die sich bei geeigneter Materialauswahl für den Mehrfachgebrauch eignet, die in einer einfachen Ausbildung jedoch insbes. auch zum einmaligen Gebrauch vorgesehen und problemlos entsorgbar ist. Die erfindungsgemäße Einrichtung eignet sich demzufolge gut zur Bestückung eines Verbandkastens, wie er bspw. gemäß Straßenverkehrsordnung in jedem Fahrzeug Vorschrift ist.

Mit der oben beschriebenen erfindungsgemäßen Beatmungseinrichtung ergibt sich der Vorteil, daß sie im Einsatz nicht auf dem Gesicht des Opfers zur Anlage kommt, sondern daß der Tubus in den Mund des zu beatmenden Opfers eingesetzt wird, bis der Opfer-Öffnungsrand des Tubus an den Zähnen bzw. am Ober- und Unterkiefer des Opfers zur Anlage kommt. Das bedeutet, daß sich diese Beatmungseinrichtung für ein einen Bart tragendes Unfallopfer genauso gut eignet wie für ein Unfallopfer, das keinen Bart trägt.

Es hat sich jedoch gezeigt, daß diese Beatmungseinrichtung bezüglich der Abdichtung des Opfer-Öffnungsrandes des Tubus noch verbesserbar ist.

Deshalb wird entsprechend einer Weiterbildung der erfindungsgemäßen Beatmungseinrichtung vorgeschlagen, daß der Opfer-Öffnungsrand umlaufend ein nachgiebiges Dichtungselement aufweist. Durch dieses entlang des Opfer-Öffnungsrandes des Tubus umlaufende, nachgiebige Dichtungselement ergibt sich im Einsatzfall der erfindungsgemäßen Beatmungseinrichtung eine zuverlässige dichte Anlage des Tubus mit seinem Opfer-Öffnungsrand bzw. mit dem dort vorgesehenen Dichtungselement an den Zähnen bzw. am Ober- und Unterkiefer des zu beatmenden Opfers, insbes. Unfallopfers, unabhängig von den anatomischen Gegebenheiten, d.h. der Kieferstruktur des Unfallopfers. Das bedeutet jedoch, daß sich die erfindungsgemäße Einrichtung ausgezeichnet zur Beatmung eines Opfers, insbes. eines Unfallopfers, eignet.

Erfindungsgemäß kann das Dichtungselement an den Opfer-Öffnungsrand des Tubus einstückig angeformt sein, wenn das zur Herstellung des Tubus vorgesehene Formwerkzeug entsprechend ausgebildet ist. Erfindungsgemäß besteht der Tubus nämlich zweckmäßigerweise aus einem geeigneten Kunststoffmaterial, bei dem es sich um ein geschlossenzelliges Kunststoff-Schaummaterial handeln kann. Anstelle einer einstückigen Ausbildung ist es jedoch auch möglich, daß bei der erfindungsgemäßen Beatmungseinrichtung das Dichtungselement von einem Dichtungswulst gebildet ist, der am Opfer-Öffnungsrand des Tubus befestigt ist. Bei einer solchen Ausbildung kann der Dichtungswulst ein Hohlprofil oder ein Vollprofil besitzen.

Um eine zuverlässige Abdichtung des Opfer-Öffnungsrandes des Tubus der erfindungsgemäßen Beatmungseinrichtung bei ihrem Gebrauch zu gewährleisten, besteht der Dichtungswulst vorzugsweise aus einem elastisch nachgiebigen Material.

Desgleichen ist es möglich, daß der Dichtungswulst aus einem plastisch nachgiebigen Material besteht. Unabhängig davon, ob ein elastisch oder plastisch nachgiebiges Material zur Anwendung gelangt, ist es bevorzugt, wenn für den Dichtungswulst ein weiches, hautfreundliches und hygienisches Material zum Einsatz gelangt.

Um ein ungewolltes bzw. plötzliches Schließen des Mundes des Opfers, insbes. des Unfallopfers, zu verhindern, ist es zweckmäßig, wenn bei der erfindungsgemäßen Einrichtung der Tubus in derNachbarschaft des Opfer-Öffnungsrandes im Bereich der Mundwinkelansätze innenseitig jeweils ein in Längsrichtung des Tubus orientiertes, sich zum Opfer-Öffnungsrand hin verjüngendes Keilelement aufweist. Jedes der beiden Keilelemente, weist dabei zweckmäßigerweise eine solche Höhe bzw. Dicke auf, daß in der Gebrauchsstellung der Beatmungseinrichtung die beiden Keilelemente zwischen der oberen und der unteren Zahnreihe des Unfallopers zu liegen kommen, so daß ein ungewolltes bzw. plötzliches Schließen des Mundes des Unfallopfers ausgeschlossen wird. Mit einer solchermaßen ausgebildeten Beatmungseinrichtung ist es also sehr zuverlässig möglich, ein Opfer, insbes. Unfallopfer, optimal zu beatmen. Demselben Zweck ist es dienlich, wenn im Inneren des Tubus eine Ventileinrichtung vorgesehen ist, die einerseits eine Luftströmung vom Helfer-Öffnungsrand zum Opfer-Öffnungsrand bei gleichzeitiger Verhinderung der Luftströme in umgekehrter Richtung vom Opfer-Öffnungsrand zum Helfer-Öffnungsrand und die andererseits eine Luftströmung vom Opfer-Öffnungsrand durch einen aus dem Tubus ausmündenden Auslaß in die Umgebung ermöglicht. Hierbei kann die Ventileinrichtung mindestens ein Filterelement aufweisen, das keimtötend und feuchtigkeitshemmend ausgebildet sein kann.

Ausführungsbeispiele der erfindungsgemäßen Einrichtung zum Beatmen, insbes. eines Unfallopfers, sind in der Zeichnung dargestellt und werden nachfolgend beschrieben. Es zeigen:
- Fig. 1: einen Längsschnitt durch eine erste Ausbildung der Einrichtung,
- Fig. 2: einen Schnitt entlang der Schnittlinie II-II in Fig.1 durch den Tubus der Einrichtung und durch das im Tubus angeordnete Filterelement,
- Fig. 3: einen Schnitt entlang der Schnittlinie III-III in Fig.1 durch das Atemrohr der Einrichtung,
- Fig. 4: einen Schnitt entlang der Schnittlinie IV-IV in Fig.1 durch das mit einem Polsterelement ausgebildete Nasenabdeckorgan,
- Fig. 5: eine Draufsicht auf eine zweite Ausbildung der Einrichtung zum Beatmen insbes. eines Unfallopfers,
- Fig. 6: einen Schnitt entlang der Schnittlinie VI-VI durch die Einrichtung gemäß Fig. 5,
- Fig. 7: einen Längsschnitt durch eine andere Ausbildung der Beatmungseinrichtung mit einer im Tubus vorgesehenen Ventileinrichtung, die in der Stellung gezeichnet ist, in welcher eine Luftströmung vom Helfer-Öffnungsrand zum Opfer-Öffnungsrand ermöglicht ist,
- Fig. 8: eine der Fig. 7 ähnliche Schnittdarstellung durch die beatmungseinrichtung gemäß Fig. 7, wobei die Ventileinrichtung in der Stellung gezeichnet ist, in welcher eine Luftströmung vom Opfer-Öffnungsrand in die Umgebung der Beatmungseinrichtung möglich ist,
- Fig. 9: einen Schnitt entlang der Schnittlinie VIII-VIII in Fig. 7, wobei auf die Darstellung der Ventileinrichtung der Ventileinrichtung verzichtet worden ist,
- Fig. 10: eine Ansicht der Ventileinrichtung allein in Blickrichtung der Pfeile X-X in Fig. 8, d.h. eine Ansicht der Ventileinrichtung in Blickrichtung vom Helfer-Öffnungsrand zum Opfer-Öffnungsrand,
- Fig. 11: drei verschiedene abschnittweise gezeichnete Ausbildungen des Opfer-Öffnungsrandes in einer Schnittdarstellung,
- Fig. 12: eine der Fig. 2 ähnliche Schnittdarstellung durch eine Ausbildung des Tubus mit Ringwülsten zum Festklemmen eines in dieser Fig. nicht gezeichneten Filterelements,
- Fig. 13: einen Schnitt entlang der Schnittlinie XIII-XIII in Fig. 12, d.h. einen Längsschnitt durch den Tubus gemäß Fig. 12,
- Fig. 14: eine der Fig. 12 ähnliche Querschnittsdarstellung einer Ausführungsform des Tubus, und
- Fig. 15: einen Längsschnitt durch den Tubus gemäß Fig. 14 entlang der Schnittlinie XV-XV in Fig. 14.

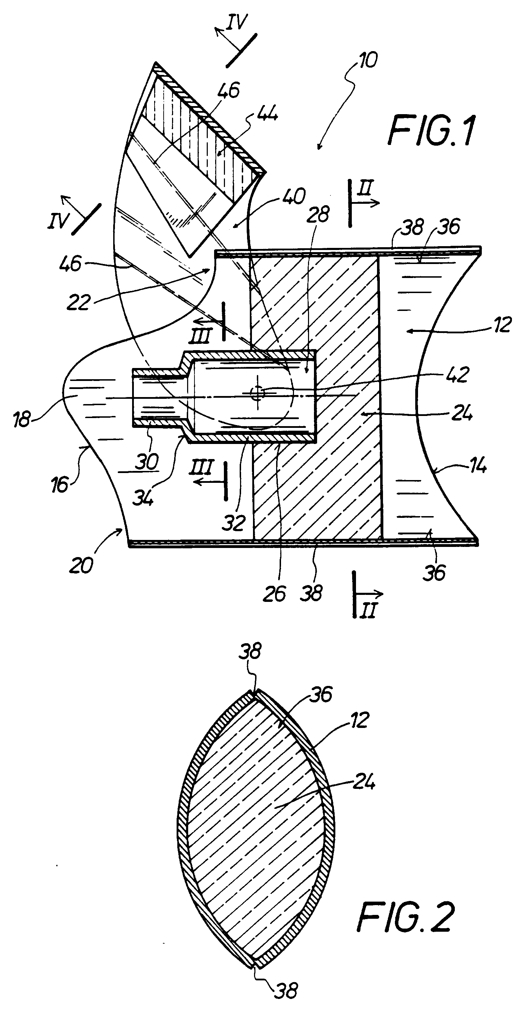

Fig. 1 zeigt längsgeschnitten eine Ausbildung der Einrichtung 10 zum Beatmen eines Unfallopfers. Die Einrichtung 10 weist einen Tubus 12 aus einem Kunststoffmaterial, aus einem Kartonmaterial o.dgl. auf. Der Öffnungsrand 14 des Tubus 12 ist konkav gekrümmt eingezogen, er bildet den Helfer-Öffnungsrand der Einrichtung 10. Der dem Öffnungsrand 14 gegenüberliegende zweite Öffnungsrand 16 des Tubus 12 ist mit zwei sich seitlich gegenüberliegenden Mundwinkel-Ansätzen 18 ausgebildet, von denen in dieser Zeichnungsfigur nur einer sichtbar ist. Der Öffnungsrand 16 bildet den Opfer-Öffnungsrand der Einrichtung 10. Der Abschnitt 20 des Opfer-Öffnungsrandes 16 ist zur Anlage am Unterkiefer des Unfallopfers und der Abschnitt 22 ist zur Anlage am Oberkiefer des Unfallopfers vorgesehen. Aus diesem Grunde sind die beiden Abschnitte 20 und 22 in axialer Richtung des Tubus gegeneinander geeignet versetzt.

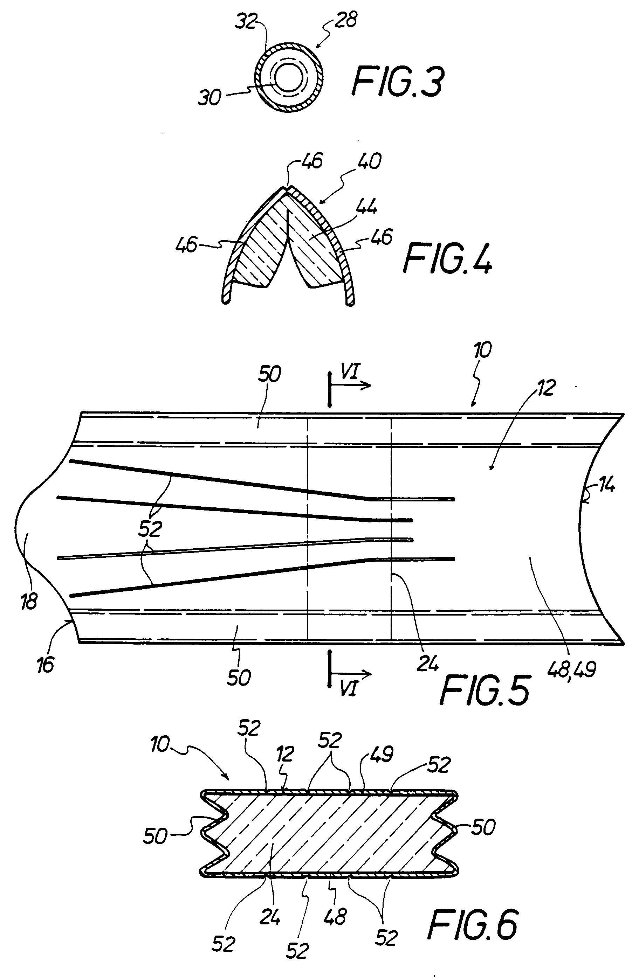

Im Inneren des Tubus 12 ist ein Filterelement 24 angeordnet, durch das ein Blut- bzw. Speichelfluß durch den Tubus 12 hindurch verhindert wird, ohne daß die Luftströmung zum Beatmen eines Unfallopfers merklich behindert wird. Das Filterelement 24 besteht zweckmäßigerweise aus einem federnd nachgiebigen, offenporigen Schaumstoffmaterial. Das Filterelement 24 ist mit einem Sackloch 26 ausgebildet, in welchem ein Atemröhrchen 28 festgelegt ist. Wie auch aus Fig. 3 ersichtlich ist, ist das Atemröhrchen 28 in axialer Richtung abgestuft ausgebildet, wobei der dem Opfer-Öffnungsrand 16 zugewandte Endabschnitt 30 einen kleineren Durchmesser besitzt als der dem Helfer-Öffnungsrand 14 zugewandte Abschnitt 32. Der Endabschnitt 30 reduzierten Durchmessers dient zum Einstecken des Atemröhrchens 28 zwischen die Zähne eines Unfallopfers, bis die Zähne an der Übergangsschulter 34 zwischen den beiden Abschnitten 30 und 32 anliegen.

Aus Fig. 2 ist ersichtlich, daß der Tubus 12 in der Lager- bzw. Ruheposition einen linsenförmigen Querschnitt aufweist, und daß das Filterelement 24 allseitig an der Innenwand 36 des Tubus 12 eng und dicht anliegt. Aus Fig. 2 sind außerdem zwei in Längsrichtung des Tubus 12 verlaufende Faltlinien 38 zu erkennen, durch welche es einfach kraftsparend und definiert möglich ist, die Einrichtung 10 platzsparend zusammenzufalten, um sie bspw. in einem Verbandkasten eines Fahrzeugs problemlos unterbringen zu können.

Fig. 1 zeigt außerdem ein Nasenabdeckorgan 40, das zweckmäßigerweise aus demselben Material besteht wie der Tubus 12 und das mit dem Tubus 12 mittels zweier seitliche Scharniere 42 schwenkbeweglich verbunden ist,wobei in Fig. 1 nur eines der beiden Scharniere 42 sichtbar ist. Wie auch aus Fig. 4 ersichtlich ist, ist das Nasenabdeckorgan 40 mit einem Polsterelement 44 ausgebildet, das aus demselben Material bestehen kann, wie das Filterelement 24 im Inneren des Tubus 12. Aus den Figuren 1 und 4 ist außerdem zu erkennen, daß das Nasenabdeckorgan 40 mit Faltlinien 46 geeignet ausgebildet ist, um es mit dem Tubus 12 platzsparend zusammenfalten und bspw. in einem Verbandkasten unterbringen zu können.

Eine andere Ausbildung der Einrichtung 10 zum Beatmen insbes. eines Unfallopfers zeigen die Figuren 5 und 6. Bei dieser Ausbildung der Einrichtung 10 ist der Tubus 12 mit einem flächigen Grundelement 48 und einem flächigen Deckelement 50 ausgebildet, die miteinander mittels ziehharmonikaförmig gefalteter Seitenabschnitte 50 verbunden sind. Der eine Öffnungsrand 14 ist auch bei dieser Ausführungsform der Einrichtung 10 konkav gekrümmt eingezogen ausgebildet; er bildet den Helfer-Öffnungsrand. Der davon entfernte zweite Öffnungsrand 16 ist auch bei dieser Ausführungsform der Einrichtung 10 mit Mundwinkel-Ansätzen 18 ausgebildet, die am Grund- und Deckelement 48 und 49 vorgesehen sind; er bildet den Opfer-Öffnungsrand.

Im Inneren des Tubus 12 ist in einem mittleren Abschnitt ein Filterelement 24 aus einem federnd nachgiebigen Schaumstoffmaterial angeordnet, das luftdurchlässig ist, einen Blut- bzw. Speichelfluß durch den Tubus 12 hindurch jedoch verhindert.

Das Grundelement 48 und/oder das Deckelement 49 des Tubus 12 ist zweckmäßigerweise mit Faltlinien 52 versehen, die insbes. dazu dienen, in der Aktivstellung der Einrichtung 10 die beiden Mundwinkel-Ansätze 18 voneinander wegzuspreizen. Auf diese Weise werden zwei Mundstücke gebildet, die zueinander senkrecht orientiert sind. Das helferseitige Mundstück ist in Fig. 5 dann in der Zeichnungsebene orientiert, während das opferseitige Mundstück zur Zeichnungsebene der Fig. 5 senkrecht orientiert ist.

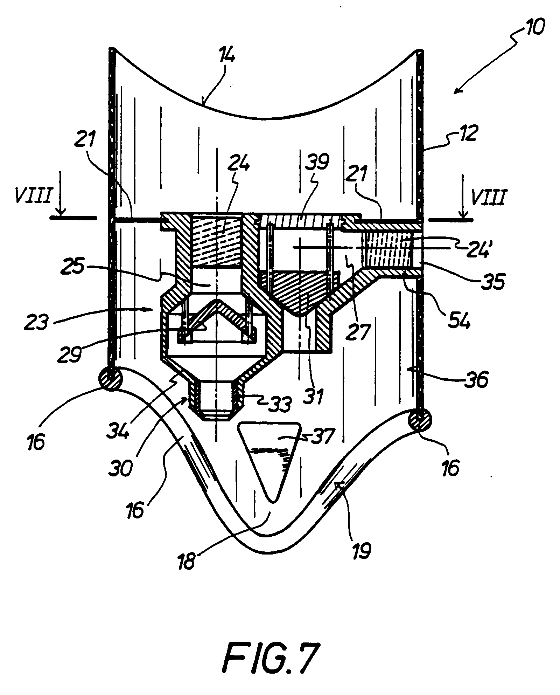

Fig. 7 zeigt eine Beatmungseinrichtung 10 mit einem in seinen Abmessungen veränderlichen, d.h. zusammendrückbaren Tubus 12. Der Tubus 12 besteht vorzugsweise aus einem geeigneten Kunststoffmaterial, er weist einen Helfer-Öffnungsrand 14 mit einer konkav gekrümmt eingezogenen Mundstückstruktur, sowie einen Opfer-Öffnungsrand 16 mit einer Mundstückstruktur auf, die zwei Mundwinkel-Ansätze 18 besitzt. Der Opfer-Öffnungsrand 16 ist mit einem nachgiebigen Dichtungselement 19 ausgebildet, das um den Opfer-Öffnungsrand 16 umläuft.

Im Inneren des Tubus 12 ist eine Zwischenwand 21 vorhanden, die zur Anbringung einer Ventileinrichtung 23 vorgesehen ist. Die Ventileinrichtung 23 weist ein Filterelement 24 in einem ersten Ventilkanal 25 sowie in einem zweiten Ventilkanal 27 ein zweites Filterelement 24' auf. Im ersten Ventilkanal 25 ist ein Ventilkörper 29 und im Ventilkanal 27 ist ein zweiter Ventilkörper 31 linear beweglich geführt vorgesehen. Der erste Ventilkanal 25 ist an seinem dem Opfer-Öffnungsrand 16 zugewandten Endabschnitt 30 mit einem sogenannten Beißröhrchen 33 versehen, durch das ein ungewolltes plötzliches Zusammenbeißen der Zähne des Opfers verhindert wird. Demselben Zweck dient die Übergangsschulter 34 des ersten Ventilkanals 25 der Ventileinrichtung 23.

Der erste Ventilkanal 25 erlaubt nur eine Luftströmung vom Helfer-Öffnungsrand 14 zum Opfer-Öffnungsrand 16, und der zweite Ventilkanal 27 erlaubt nur eine Luftströmung vom Opfer-Öffnungsrand in die Umgebung des Tubus 12 bzw. der Beatmungseinrichtung 10. Zu diesem Zweck ist der Tubus 12 seitlich mit einer Auslaßöffnung 35 ausgebildet.

Der Tubus 12 ist an seiner Innenseite 36 im Bereich seiner beiden Mundwinkel-Ansätze 18 jeweils mit einem Keilelement 37 ausgebildet, das in Längsrichtung des Tubus 12 orientiert und zum Opfer-Öffnungsrand 16 hin verjüngt ist.

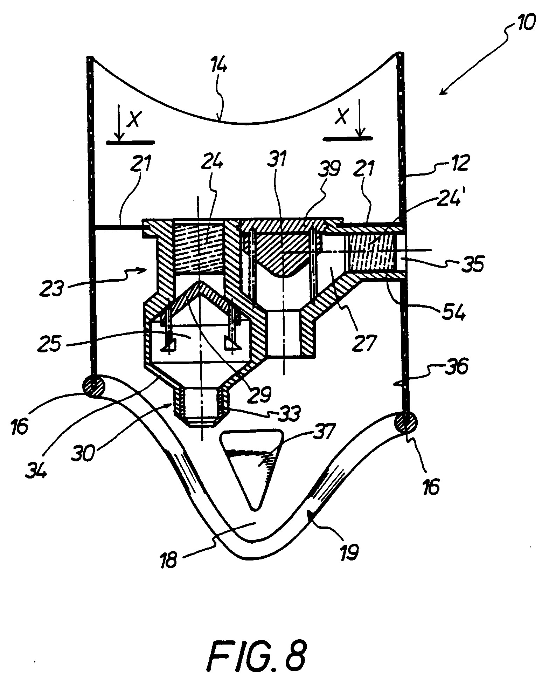

Die Fig. 7 zeigt die Beatmungseinrichtung 10 in der Stellung, in welcher zwischen dem Helfer-Öffnungsrand 14 durch den ersten Ventilkanal 25 der Ventileinrichtung 23 hindurch zum Opfer-Öffnungsrand 16 eine fluidische Verbindung gegeben ist, während gleichzeitig der zweite Ventilkanal 27 zwischen dem Opfer-Öffnungsrand 16 und der Umgebung bzw. der Auslaßöffnung 35 im Tubus 12 verschlossen ist. Demgegenüber zeigt Fig. 8 die Beatmungseinrichtung 10 gem. Fig. 7 in der Stellung, in welcher der erste Ventilkanal 25 abgedichtet und der zweite Ventilkanal 27 geöffnet ist. Gleiche Einzelheiten sind in Fig. 8 mit denselben Bezugsziffern wie in Fig. 7 bezeichnet, so daß es sich erübrigt, in Verbindung mit Fig. 8 alle diese Einzelheiten noch einmal detailliert zu beschreiben. Die in Fig. 8 gezeichnete Betriebsstellung der Beatmungseinrichtung 10 ist gegeben, wenn das beatmete Opfer selbst zu atmen beginnt.

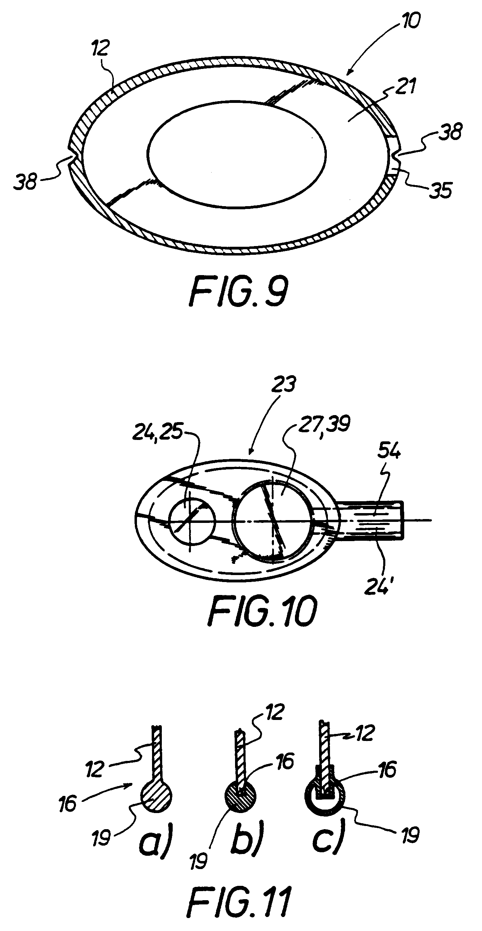

Fig. 9 zeigt einen Querschnitt durch den Tubus 12 der Beatmungseinrichtung 10 zur Verdeutlichung der Zwischenwand 21, die zur Festlegung der Ventileinrichtung 232 (sh. die Figuren 7 und 8) vorgesehen ist. Außerdem ist aus Fig. 9 die im Tubus 12 ausgebildete Auslaßöffnung 35 zu erkennen. Desweiteren sind in Fig. 9 zwei Faltlinien 38 verdeutlicht, die in Längsrichtung des Tubus 12 verlaufen, um den Tubus 12 zusammenfaltbar zu gestalten.

Fig. 10 zeigt in einer Ansicht von oben die Ventileinrichtung 23 mit dem im ersten Ventilkanal 25 angeordneten Filterelement 24, sowie einen Deckel 39, der herstellungsbedingt vorgesehen ist und zur Begrenzung des zweiten Ventilkanals 27 dient, wie auch aus den Figuren 7 und 8 ersichtlich ist. Die Ventileinrichtung 23 ist mit einem seitlich wegstehenden Rohrabschnitt 54 ausgebildet, in welchem das Filterelement 24' des zweiten Ventilkanals 27 angeordnet ist (sh. auch Figuren 7 und 8).

Die Fig. 11 zeigt drei verschiedene Möglichkeiten der Ausbildung des Opfer-Öffnungsrandes 16, wobei in der Fig. 11a eine Ausbildung verdeutlicht ist, bei welcher das Dichtungselement 19 an den Tubus 12 bzw. dessen Opfer-Öffnungsrand 16 einstückig angeformt ist. Die Fig. 11b zeigt eine Ausführungsform, in welcher am Opfer-Öffnungsrand 16 des Tubus 12 ein selbständiges Dichtungselement 19 befestigt ist. Diese Befestigung kann durch Kleben, Schweißen o.dgl. erfolgen. Während in Fig. 11b ein Dichtungswulst 19 mit einem Vollprofil verdeutlicht ist, zeigt die Fig. 11c ein Dichtungselement 19, das von einem Dichtungswulst mit einem Hohlprofil gebildet ist.

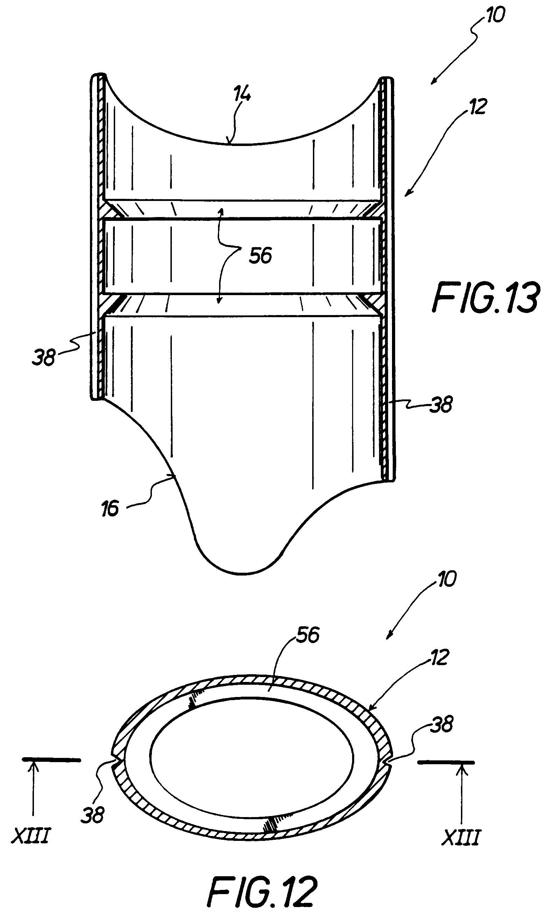

Die Fig. 12 und 13 zeigen in einem Querschnitt und in einem Längsschnitt eine Ausbildung des Tubus 12 der Beatmungseinrichtung 10 wobei auf die Darstellung des im Tubus 12 angeordneten Filterelementes verzichtet worden ist. Der Tubus 12 ist in seinem Inneren mit zwei Ringwülsten 56 ausgebildet, die zum zuverlässigen Festklemmen des (nicht gezeichneten) Filterelementes dienen. Zu diesem Zwecke ist der axiale Abstand der beiden Ringwülste 56 voneinander an die Dicke des zwischen ihnen festzuklemmenden Filterelementes angepaßt.

Die Ringwülste 56 sind mit dem Tubus 12 zweckmäßigerweise einstückig ausgebildet.

Mit der Bezugsziffer 14 ist in Fig. 13 der Helfer-Öffnungsrand und mit der Bezugsziffer 16 ist in dieser Zeichnungsfigur der Opfer-Öffnungsrand bezeichnet.

Um den Tubus 12 platzsparend zusammenfalten zu können, ist er mit Faltlinien 38 ausgebildet, die in Längsrichtung des Tubus 12 verlaufen.

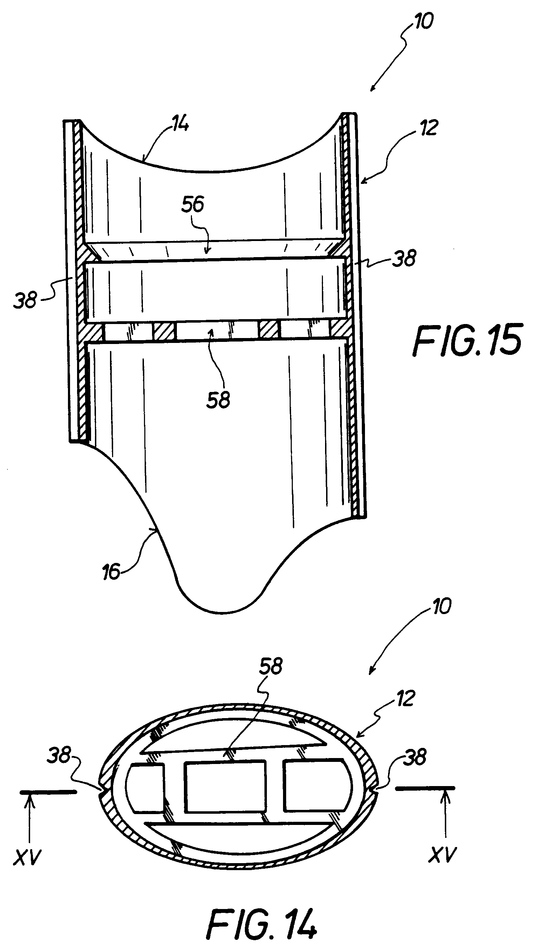

Die Fig. 14 und 15 zeigen eine Ausbildung des Tubus 12 der Beatmungseinrichtung 10, der in seinem Inneren ein den Tubus 12 querendes und vorzugsweise zusammenfaltbares Gitterelement 58 sowie einen vom Gitterelement 58 in Längsrichtung des Tubus 12 beabstandeten Ringwulst 56 aufweist. Das Gitterelement 58 ist hierbei auf der dem Opfer-Öffnungsrand 16 zugewandten Seite des Tubus 12 und der Ringwulst 56 ist auf der dem Helfer-Öffnungsrand 14 des Tubus 12 zugewandten Seite vorgesehen. Der Abstand zwischen dem Gitterelement 58 und dem Ringwulst 56 ist an die Dicke des (nicht dargestellten) Filterelementes angepaßt, um das besagte Filterelement im Tubus 12 der Beatmungseinrichtung 10 zuverlässig festzuklemmen.

Mit der Bezugsziffer 38 sind auch in den Fig. 14 und 15 in Längsrichtung des Tubus 12 orientierte Faltlinien bezeichnet, um den Tubus 12 platzsparend zusammenfalten zu können.

## Patentansprüche

1. Einrichtung zum Beatmen eines Unfallopfers, mit einem in seinen Abmessungen veränderlichen Tubus (12), der an seinen beiden Öffnungsrändern (14, 16) mit jeweils einem Mundstück für einen Helfer und für das zu beatmende Unfallopfer ausgebildet ist, wobei die Öffnungsränder (14, 16) des Tubus (121) derartig gestaltet sind, daß sie im Aktivzustand der Einrichtung (10) zueinander orientierte Mundstücke bilden, wobei der Helfer-Öffnungsrand (14) des Tubus (12) eine konkav gekrümmt eingezogene Mundstückstruktur und der Opfer-Öffnungsrand (16) des Tubus (12) eine Mundstückstruktur mit zwei Mundwinkel-Ansätzen (18) aufweist,
**dadurch gekennzeichnet,**
daß der Tubus (12) durch in seiner Längsrichtung verlaufende Faltlinien (38; 52) zusammenfaltbar ausgebildet ist, und daß im Inneren des Tubus (12) ein Filterelement (24) vorgesehen ist, das aus einem luftdurchlässigen, elastisch nachgiebigen Schaumstoffmaterial besteht.

2. Einrichtung nach Anspruch 1,
**dadurch gekennzeichnet,**
daß der Tubus (12) in seinem Inneren mit einer Halterung (56, 58) für das Filterelement (24) ausgebildet ist.

3. Einrichtung nach Anspruch 2,
**dadurch gekennzeichnet,**
daß die Halterung (56, 58) von zwei Ringwülsten (56) gebildet ist, die in Längsrichtung des Tubus (12) voneinander beabstandet sind.

4. Eine Einrichtung nach Anspruch 2,
**dadurch gekennzeichnet,**
daß die Halterung (56, 58) von einem den Tubus (12) querenden Gitterelement (58) und einem davon in Längsrichtung des Tubus (12) beabstandeten Ringwulst (56) gebildet ist, wobei das Gitterelement (58) auf der dem Opfer-Öffnungsrand (16) zugewandten Seite und der Ringwulst (56) auf der dem Helfer-Öffnungsrand (14) zugewandten Seite des Tubus (12) vorgesehen ist.

5. Einrichtung nach einem der vorhergehenden Ansprüche,
**dadurch gekennzeichnet,**
daß das Filterelement (24) ein Atemröhrchen (28) aufweist, das vom Filterelement (24) zum Opfer-Öffnungsrand (16) weist.

6. Einrichtung nach einem der vorhergehenden Ansprüche,
**dadurch gekennzeichnet,**
daß der Tubus (12) mit einem Nasenabdeckorgan (40) versehen ist.

7. Einrichtung nach Anspruch 6
**dadurch gekennzeichnet,**
daß das Nasenabdeckorgan (40) mit einem Polsterelement (44) ausgebildet ist.

8. Einrichtung nach Anspruch 6 oder 7,
**dadurch gekennzeichnet,**
daß das Nasenabdeckorgan (40) an den Tubus (12) einteilig angeformt ist.

9. Einrichtung nach Anspruch 6 oder 7,
**dadurch gekennzeichnet,**
daß das Nasenabdeckorgan (40) mit dem Tubus (12) schwenkbeweglich verbunden ist.

10. Einrichtung nach einem der Ansprüche 6 bis 9,
**dadurch gekennzeichnet,**
daß das Nasenabdeckorgan (40) mit Faltlinien (46) ausgebildet ist.

11. Einrichtung nach einem der vorhergehenden Ansprüche,
**dadurch gekennzeichnet,**
daß der Opfer-Öffnungsrand (16) umlaufend ein nachgiebiges Dichtungselement (19) aufweist.

12. Einrichtung nach Anspruch 11,
**dadurch gekennzeichnet,**
daß das Dichtungselement (19) an den Opfer-Öffnungsrand (16) des Tubus (12) einstückig angeformt ist.

13. Einrichtung nach Anspruch 11,
**dadurch gekennzeichnet,**
daß das Dichtungselement (19) von einem Dichtungswulst gebildet ist, der am Opfer-Öffnungsrand (16) des Tubus (12) befestigt ist.

14. Einrichtung nach Anspruch 13,
**dadurch gekennzeichnet,**
daß der Dichtungswulst ein Hohlprofil besitzt.

15. Einrichtung nach Anspruch 13,
**dadurch gekennzeichnet,**
daß der Dichtungswulst ein Vollprofil besitzt.

16. Einrichtung nach einem der Ansprüche 13 bis 15,
**dadurch gekennzeichnet,**
daß der Dichtungswulst aus einem elastisch nachgiebigen Material besteht.

17. Einrichtung nach einem der Ansprüche 13 bis 15,
**dadurch gekennzeichnet,**
daß der Dichtungswulst aus einem plastisch nachgiebigen Material besteht.

18. Einrichtung nach einem der vorhergehenden Ansprüche,
**dadurch gekennzeichnet,**
daß der Tubus (12) in der Nachbarschaft des Opfer-Öffnungsrandes (16) im Bereich der Mundwinkel-Ansätze (18) innenseitig jeweils ein in Längsrichtung des Tubus (12) orientiertes, sich zum Opfer-Öffnungsrand (16) hin verjüngendes Keilelement (37) aufweist.

19. Einrichtung nach einem der vorhergehenden Ansprüche,
**dadurch gekennzeichnet,**
daß das Innere des Tubus (12) von einer Zwischenwand (21) überspannt ist, in der eine Ventileinrichtung (23) angeordnet ist, die einerseits eine Luftströmung vom Helfer-Öffnungsrand zum Opfer-Öffnungsrand (16) bei gleichzeitiger Verhinderung einer Luftströmung in umgekehrter Richtung vom Opfer-Öffnungsrand (16) zum Helfer-Öffnungsrand (14) und die andererseits eine Luftströmung vom Opfer-Öffnungsrand (16) durch einen aus dem Tubus (12) ausmündenden Auslaß (35) in die Umgebung ermöglicht.

20. Einrichtung nach Anspruch 19,
**dadurch gekennzeichnet,**
daß die Ventileinrichtung (23) mindestens ein Filterelement (24, 24') aufweist.

## Claims

1. Appliance for giving artificial respiration to a casualty, with a tube (12) the dimensions of which are variable and which is designed at its two orifice edges (14, 16) with a mouthpiece respectively for a helper and for the casualty to be given artificial respiration, the orifice edges (14, 16) of the tube (12) being shaped in such a way that when the appliance (10) is in the active state they form mouthpieces oriented with respect to one another, the helper's orifice edge (14) of the tube (12) having a mouthpiece structure drawn in in a concavely curved manner, and the casualty's orifice edge (16) of the tube (12) having a mouthpiece structure with two mouth-corner extensions (18); characterized in that the tube (12) is designed so as to be capable of being folded together by means of folding lines (38; 52) running in its longitudinal direction; and in that there is provided inside the tube (12) a filter element (24) which consists of an air-permeable, elastically flexible foam material.

2. Appliance according to Claim 1, characterized in that the tube (12) is designed, inside it, with a holding device (56, 58) of the filter element (24).

3. Appliance according to Claim 2, characterized in that the holding device (56, 58) is formed by two annular beads (56) which are spaced from one another in the longitudinal direction of the tube (12).

4. Appliance according to Claim 2, characterized in that the holding device (56, 58) is formed by a grid element (58) crossing the tube (12) and by an annular bead (56) spaced from the said grid element in the longitudinal direction of the tube (12), the grid element (58) being provided on that side of the tube (12) which faces the casualty's orifice edge (16), and the annular bead (56) being provided on that side of the tube (12) which faces the helper's orifice edge (14).

5. Appliance according to one of the preceding claims, characterized in that the filter element (24) has a small breathing tube (28) which points from the filter element (24) towards the casualty's orifice edge (16).

6. Appliance according to one of the preceding claims, characterized in that the tube (12) is provided with a nose-covering member (40).

7. Appliance according to Claim 6, characterized in that the nose-covering member (40) is designed with a padding element (44).

8. Appliance according to Claim 6 or 7, characterized in that the nose-covering member (40) is formed in one piece on the tube (12).

9. Appliance according to Claim 6 or 7, characterized in that the nose-covering member (40) is pivotably connected to the tube (12).

10. Appliance according to one of Claims 6 to 9, characterized in that the nose-covering member (40) is designed with folding lines (46).

11. Appliance according to one of the preceding claims, characterized in that the casualty's orifice edge (16) has peripherally a flexible sealing element (19).

12. Appliance according to Claim 11, characterized in that the sealing element (19) is formed in one piece on the casualty's orifice edge (16) of the tube (12).

13. Appliance according to Claim 11, characterized in that the sealing element (19) is formed by a sealing bead which is fastened to the casualty's orifice edge (16) of the tube (12).

14. Appliance according to Claim 13, characterized in that the sealing bead possesses a hollow profile.

15. Appliance according to Claim 13, characterized in that the sealing bead possesses a solid profile.

16. Appliance according to one of Claims 13 to 15, characterized in that the sealing bead consists of an elastically flexible material.

17. Appliance according to one of Claims 13 to 15, characterized in that the sealing bead consists of a plastically flexible material.

18. Appliance according to one of the preceding claims, characterized in that, in the vicinity of the casualty's orifice edge (16), the tube (12) has on the inside, in the region of each of the mouth-corner extensions (18), a wedge element (37) oriented in the longitudinal direction of the tube (12) and tapering towards the casualty's orifice edge (16).

19. Appliance according to one of the preceding claims, characterized in that the interior of the tube (12) is spanned by an intermediate wall (21), in which a valve device (23) is arranged which, on the one hand, allows an air flow from the helper's orifice edge to the casualty's orifice edge (16) whilst at the same time preventing an air flow in the opposite direction from the casualty's orifice edge (16) to the helper's orifice edge (14), and, on the other hand, allows an air flow from the casualty's orifice edge (16) through an outlet (35) opening out of the tube (12), into the environment.

20. Appliance according to Claim 19, characterized in that the valve device (23) has at least one filter element (24, 24').

## Revendications

1. Dispositif pour rétablir la respiration d'une victime d'un accident au moyen d'une tubulure (12) de dimensions variables, qui comporte aux bords de chacune de ses deux ouvertures (14, 16) une pièce d'embouchure pour un secouriste et pour la victime d'accident, les bords des ouvertures (14, 16) de la tubulure (12) étant conformés de manière telle que, dans l'état actif du dispositif (10), ils constituent des pièces d'embouchure orientées l'une vers l'autre, le bord de l'ouverture (14) de la tubulure (12) destinée au secouriste présentant une structure de pièce d'embouchure rentrée de façon concave et le bord de l'ouverture (16) de la tubulure (12) destinée à la victime présentant une structure de pièce d'embouchure comportant deux appendices s'ouvrant angulairement (18),
caractérisé en ce que la tubulure (12) présente dans sa direction longitudinale des lignes de pliage (38, 52) repliables ensemble, et un élément de filtration (24) est prévu, constitué d'un matériau en mousse élastiquement souple, laissant passer l'air.

2. Dispositif selon la revendication 1, caractérisé en ce que la tubulure (12) comporte intérieurement des supports (56, 58) pour l'élément de filtration.

3. Dispositif selon la revendication 2, caractérisé en ce que les supports (56, 58) sont constitués de deux bourrelets annulaires (56) qui sont séparés l'un de l'autre dans la direction longitudinale de la tubulure (12).

4. Dispositif selon la revendication 2, caractérisé en ce que les supports (56, 58) sont constitués d'un élément de grille (58) transversal à la tubulure (12) et d'un bourrelet annulaire (56) séparé du précédent dans la direction longitudinale de la tubulure (12), l'élément de grille (58) étant prévu du côté orienté vers le bord de l'ouverture (16) destinée à la victime et le bourrelet annulaire (56) étant prévu du côté de la tubulure (12) orienté vers le bord de l'ouverture (14) destinée au secouriste.

5. Dispositif selon l'une des revendications précédentes, caractérisé en ce que l'élément de filtration (24) présente une canule de respiration (28) allant de l'élément de filtration (24) vers le bord de l'ouverture (16) destinée à la victime.

6. Dispositif selon l'une des revendications précédentes, caractérisé en ce que la tubulure (12) est équipée d'un organe de recouvrement du nez (40).

7. Dispositif selon la revendication 6, caractérisé en ce que l'organe de recouvrement du nez (40) comporte un élément capitonné (44).

8. Dispositif selon les revendications 6 ou 7, caractérisé en ce que l'organe de recouvrement du nez (40) est créé en une seule pièce avec la tubulure (12).

9. Dispositif selon les revendications 6 ou 7, caractérisé en ce que l'organe de recouvrement du nez (40) est réuni mobile en basculement avec la tubulure (12).

10. Dispositif selon l'une des revendications 6 à 9, caractérisé en ce que l'organe de recouvrement du nez comporte des lignes de plage (46).

11. Dispositif selon l'une des revendications précédentes, caractérisé en ce que le bord de l'ouverture (16) destinée à la victime présente sur sa périphérie un élément d'étanchéité souple (19).

12. Dispositif selon la revendication 11, caractérisé en ce que l'élément d'étanchéité (19) est conformé en une seule pièce avec le bord de l'ouverture (16) de la tubulure (12) destinée à la victime.

13. Dispositif selon la revendication 11, caractérisé en ce que l'élément d'étanchéité (19) constitue un bourrelet d'étanchéité qui est fixé au bord de l'ouverture (16) de la tubulure (12) destinée à la victime.

14. Dispositif selon la revendication 13, caractérisé en ce que le bourrelet d'étanchéité présente un profil creux.

15. Dispositif selon la revendication 13, caractérisé en ce que le bourrelet d'étanchéité présente un profil plein.

16. Dispositif selon l'une des revendications 13 à 15, caractérisé en ce que le bourrelet d'étanchéité est constitué en un matériau élastique souple.

17. Dispositif selon l'une des revendications 13 à 15, caractérisé en ce que le bourrelet d'étanchéité est constitué en un matériau plastique souple.

18. Dispositif selon l'une des revendications précédentes, caractérisé en ce que la tubulure (12), au voisinage du bord de l'ouverture (16) destinée à la victime, dans la zone des appendices s'ouvrant angulairement (18), présente intérieurement un élément orienté dans la direction longitudinale de la tubulure (12) et se rétrécissant en forme de coin vers le bord de l'ouverture destinée à la victime.

19. Dispositif selon l'une des revendications précédentes, caractérisé en ce que l'intérieur de la tubulure (12) est maintenu tendu par une paroi intermédiaire (21) dans laquelle est disposé un dispositif de ventilation (23) qui rend possible d'une part une circulation d'air depuis le bord de l'ouverture destinée au secouriste vers le bord de l'ouverture (16) destinée à la victime tout en empêchant en même temps une circulation en sens inverse, depuis le bord de l'ouverture (16) destinée à la victime vers le bord de l'ouverture (14) destinée au secouriste et, d'autre part, une circulation d'air depuis le bord de l'ouverture (16) destinée à la victime vers l'environnement par une sortie (35) s'ouvrant dans la tubulure (12).

20. Dispositif selon la revendication 19, caractérisé en ce que le dispositif de ventilation (23) comporte au moins un élément de filtration (24, 24').
